# EUROPÄISCHE PATENTSCHRIFT

(11) **EP 3 640 238 B1**
(45) Veröffentlichungstag und Bekanntmachung des Hinweises auf die Patenterteilung: **15.09.2021**
(21) Anmeldenummer: 19202626.8
(22) Anmeldetag: 11.10.2019
(51) Int. Cl.: C07C 209/78, C07C 211/50

(54) **VERFAHREN ZUR HERSTELLUNG VON DI- UND POLYAMINEN DER DIPHENYLMETHANREIHE**
METHOD FOR THE PREPARATION OF DI- AND POLYAMINES OF THE DIPHENYL METHANE SERIES
PROCÉDÉ DE PRODUCTION DE DI- ET POLYAMINES DE LA SÉRIE DU DIPHÉNYLMÉTHANE

(30) Priorität: 17.10.2018 EP 18200855
(43) Veröffentlichungstag der Anmeldung: 22.04.2020
(73) Patentinhaber: Covestro Deutschland AG, 51373 Leverkusen (DE)
(72) Erfinder: WERSHOFEN, Stefan, 41065 Mönchengladbach (DE); ADAMSON, Richard, 42799 Leichlingen (DE); PIRKL, Georg, 51375 Leverkusen (DE)
(74) Vertreter: Levpat

(56) Entgegenhaltungen:
- WO-A1-2006/103189

## Beschreibung

Die Erfindung betrifft ein Verfahren zur Herstellung von Di- und Polyaminen der Diphenylmethanreihe (MDA), bei dem zunächst Roh-MDA hergestellt und dieses dann destillativ aufgearbeitet wird, wobei die destillative Aufarbeitung Folgendes umfasst:
- In einer Destillationskolonne, Abtrennen eines Anilin und Wasser enthaltenden Stroms als Kopfprodukt unter Erhalt des ersten Produktstroms als Sumpfprodukt,
- in dieser Destillationskolonne oder in einer stromabwärts zu dieser Destillationskolonne angeordneten Vorrichtung, Strippen mit Wasserdampf unter Erhalt eines gasförmigen Anilin, Wasser und MMDA enthaltenden Stroms,
- partielles Kondensieren dieses gasförmigen, Anilin, Wasser und MMDA enthaltenden Stroms unter Erhalt eines MMDA und Wasser (sowie gegebenenfalls Anilin) enthaltenden flüssigen Stroms und eines gasförmigen Stroms enthaltend Anilin und Wasser (sowie gegebenenfalls MMDA),
- Trocknen des durch das partielle Kondensieren erhaltenen MMDA und Wasser (sowie gegebenenfalls Anilin) enthaltenden flüssigen Stroms unter Erhalt des zweiten Produktstroms.

Di- und Polyamine der Diphenylmethanreihe sind wichtige Ausgangstoffe für die Herstellung der korrespondierenden Isocyanate. Diese wiederum werden in großen Mengen hergestellt und insbesondere zur Herstellung von Polyurethanen eingesetzt. Die großtechnische Herstellung dieser Isocyanate ist in der Literatur vielfach beschrieben und erfolgt insbesondere durch Umsetzung der korrespondierenden Amine mit Phosgen in einem Lösungsmittel. Im Sinne der vorliegenden Erfindung werden unter Di- und Polyaminen der Diphenylmethanreihe Amine und Gemische von Aminen folgenden Typs verstanden:

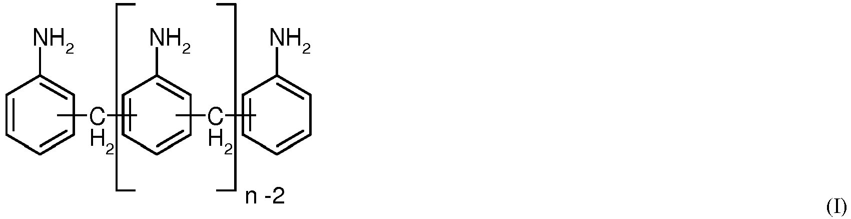

Dabei steht n für eine natürliche Zahl ≥ 2. Im Folgenden werden die Verbindungen dieses Typs, bei denen n = 2 ist, als *Diamine der Diphenylmethanreihe* oder *Diaminodiphenylmethane* (nachfolgend MMDA) bezeichnet. Verbindungen dieses Typs, bei denen n > 2 ist, werden im Rahmen dieser Erfindung als *Polyamine der Diphenylmethanreihe* oder *Polyphenylenpolymethylenpolyamine* (nachfolgend PMDA) bezeichnet. Gemische aus beiden Typen werden als *Di- und Polyamine der Diphenylmethanreihe* bezeichnet (nachfolgend MDA). Die korrespondierenden Isocyanate, die sich formal durch Ersatz aller NH₂-Gruppen durch NCO-Gruppen aus den Verbindungen der Formel (I) ableiten lassen, werden dementsprechend als *Diisocyanate der Diphenylmethanreihe* (nachfolgend MMDI), *Polyisocyanate der Diphenylmethanreihe* oder *Polyphenylenpolymethylenpolyisocyanate* (nachfolgend PMDI) bzw. *Di- und Polyisocyanate der Diphenylmethanreihe* (nachfolgend MDI) bezeichnet. Das Polymere (n > 2) liegt dabei sowohl beim Amin als auch beim Isocyanat in der Regel immer im Gemisch mit der difunktionellen Verbindung (n = 2) vor, so dass in der Praxis nur zwei Verbindungstypen relevant sind, die reinen Diamine bzw. Diisocyanate (MMDA bzw. MMDI) auf der einen Seite und das Gemisch aus difunktioneller Verbindung und dem Polymeren (MDA bzw. MDI) auf der anderen Seite. Dabei werden im Rahmen der vorliegenden Erfindung Ströme der Amine bzw. Isocyanate als "reine" difunktionelle Verbindungen (MMDA bzw. MMDI) bezeichnet, wenn der Massenanteil der difunktionelle Verbindungen, bezogen auf die Gesamtmasse des jeweiligen Stroms, mindestens 95,0 % beträgt.

MDA wird üblicherweise durch sauer katalysierte Umsetzung von Anilin mit Formaldehyd mit nachfolgender Neutralisation und Aufarbeitung des Umsetzungsprodukts hergestellt. Die kontinuierliche, diskontinuierliche oder halbkontinuierliche Herstellung von Di- und Polyaminen der Diphenylmethanreihe ist in zahlreichen Publikationen und Patenten beschrieben (beispielsweise EP-A-31423; EP 934 922 B1; EP-B-1 167 343; EP-A-1 403 242; EP-A-1 707 557; EP-A-1 813 597; EP-A-1 813 598; US-A-5,310,769; DE-A-198 04 918; JP-A-2004026753). Das als Umsetzungsprodukt erhaltene MDA enthält im Wesentlichen MMDA (als Gemisch der drei technisch bedeutsamen MMDA-Isomeren 4,4'-Diaminodiphenylmethan, 2,4'-Diaminodiphenylmethan und 2,2'-Diaminodiphenylmethan) sowie die entsprechenden höherkernigen PMDA-Homologen und -Isomeren. Daneben sind im MDA noch zahlreiche Nebenprodukte und Spurenkomponenten in stark unterschiedlichen Anteilen enthalten. Insbesondere enthält rohes MDA regelmäßig nicht umgesetztes Anilin und Wasser. Deren Abtrennung auf weniger als 1000 ppm (Anilin) bzw. weniger als 200 ppm (Wasser) durch ein mindestens zweistufiges Destillationsverfahren umfassend eine Entspannungsverdampfung und anschließende Kühlung ist in EP-A-1 813 597 beschrieben.

Hauptverwendungszweck von MDA ist die bereits erwähnte Herstellung des entsprechenden Isocyanats. In den meisten großtechnischen Verfahren wird das MDA so, wie es in der Säurekatalysierten Kondensation von Anilin und Formaldehyd erhalten wird, d. h. ohne Auftrennung in seine Isomeren bzw. Homologen, direkt weiter zu MDI phosgeniert. Erst auf der Isocyanat-Stufe erfolgt eine Auftrennung in das Diisocyanat (MMDI) und ein Gemisch aus MMDI und PMDI, dessen MMDI-Anteil entsprechend der abgetrennten Menge des Diisocyanats gegenüber dem Rohprodukt verringert ist.

Für bestimmte Einsatzgebiete, beispielsweise als Vernetzer in Kunststoffen oder Lacken, kann auch das Amin selbst eingesetzt werden, sei es in Form des bereits beschriebenen Isomeren- und Homologengemisches (MDA), sei es in Form des Diamins (MMDA). Darüber hinaus ist auch die Kernhydrierung, insbesondere zur Herstellung des kernhydrierten Diamins (H₁₂-MMDA, Diaminodicyclohexylmethan), bekannt (siehe z. B. WO 2009/144148 A1, WO 2008/077672 A1). H₁₂-MMDA kann mittels Phosgenierung oder über alternative Verfahren seinerseits in das entsprechende Isocyanat überführt werden (H₁₂-MMDI, Dicyclohexylmethandiisocyanat) (siehe z. B. WO 2009/144148 A1, WO 2008/077672 A1). Für viele solche nicht die Herstellung von MDI betreffenden Anwendungsgebiete ist es wünschenswert, das Diamin (MMDA) in möglichst großer Reinheit, d. h. mit möglichst geringem Anteil an Polymeren, zur Verfügung zu haben.

Daneben kann reines MMDA im Gegensatz zu MDA (das in der Terminologie der vorliegenden Erfindung immer substanzielle Anteile an schwer verdampfbaren höheren Homologen enthält) problemlos *in der Gasphase* zu MMDI phosgeniert werden, sodass es möglich wird, sich die aus der Herstellung von Toluylendiisocyanat (TDI) bekannten Vorteile der Gasphasenphosgenierung zu Nutze zu machen.

Aus dem Stand der Technik ist eine Reihe von Verfahren zur Abtrennung von MMDA aus MDA bekannt:
So kann die Abtrennung von MMDA und Reindarstellung des 4,4'-MMDA mittels Extraktion, wie beispielsweise in SU 463 658 beschrieben, mittels Umsetzung mit Metallsalzen, wie in GB 1 169 127 beschrieben, durch Schmelzen, wie in EP-A-0 572 030 beschrieben, oder durch Behandlung mit Lösungsmitteln, wie in BE 855 402 und US 4,034,039 beschrieben, erfolgen.

RO 104327 B1 beschreibt die Abtrennung von MMDA mittels Dünnschichtdestillation. Es ist weiterhin bekannt, MMDA mittels Destillation aus MDA abzutrennen.

DE-OS-1 901 993 beschreibt ein Verfahren zur Herstellung von 4,4'-MMDA, bei dem aus einem Gemisch aus MMDA und PMDA das MMDA abdestilliert und daraus durch Kristallisation 4,4'-MMDA abgetrennt wird. Die Destillation erfolgt bei 2 Torr und 220 bis 230 °C.

In DE-OS-100 31 540 wird ein Verfahren zur Abtrennung von 2,2'-MMDA und 2,4'-MMDA aus MDA beschrieben. Dazu kann eine Destillationskolonne mit mindestens 40 Trennstufen eingesetzt werden. Die Destillation erfolgt bei einem Temperaturverlauf von 180 bis 280 °C, einem Kopfdruck von 0,1 bis 10 mbar und einem Sumpfdruck von 8 bis 20 mbar. Zur Reduzierung der Druckverluste werden druckverlustarme Gewebepackungen eingesetzt. Das von 2,2'- und 2,4'-MMDA befreite MDA wird mit Phosgen zu MDI umgesetzt, das abgetrennte 2,2'- und 2,4'-MMDA wird in die Kondensationsstufe zurückgeführt.

WO 2006/103189 A1 beschreibt die Herstellung von MDA, dessen Auftrennung in einen im Wesentlichen MMDA enthaltenden Teilstrom und einen das verbleibende MMDA und PMDA enthaltenden Teilstrom und getrennte Phosgenierung der beiden Teilströme. Der im Wesentlichen MMDA enthaltende Teilstrom wird in der Gasphase und der das verbleibende MMDA und PMDA enthaltende Teilstrom in der Flüssigphase phosgeniert. Die Auftrennung der beiden Amin-Teilströme erfolgt vorzugsweise destillativ, z. B. in zwei aufeinanderfolgenden Destillationskolonnen oder in einer Trennwandkolonne, wobei bei der letztgenannten Ausführungsform der im Wesentlichen aus MMDA bestehende Teilstrom in einem Seitenabzug der Trennwandkolonne entnommen wird.

Eine destillative Abtrennung von 4,4'-MMDA wird in WO 2007/085534 A1 beschrieben, bei der MDA in zwei Teilströme aufgeteilt wird. Der erste Teilstrom wird z. B. in zwei aufeinanderfolgenden Kolonnen oder in einer Trennwandkolonne in einen Hauptstrom enthaltend im Wesentlichen 4,4'-MMDA, einen Sumpfstrom und einen Kopfstrom aufgeteilt. Sumpfstrom und Kopfstrom werden mit dem zweiten Teilstrom wieder vereinigt und z. B. als Ausgangsmaterial für eine Phosgenierung zu MDI genutzt.

Nachteilig bei allen bisherigen Verfahren zur Abtrennung von MMDA und/oder der Trennung der MMDA-Isomeren ist es, dass dazu ein apparativ aufwendiges separates Verfahren nötig ist (z. B. zweistufige Destillation oder der Einsatz einer regeltechnisch schwer beherrschbaren Trennwandkolonne), bei dem die anfallenden Nebenprodukte zumeist entsorgt werden müssen. Darüber hinaus kann der Einsatz z. B. einer separaten Destillationsstufe aufgrund der erhöhten thermischen Belastung zu einer Beeinträchtigung der Qualität des erhaltenen MDA und des daraus hergestellten MDI führen.

Es bestand daher ein Bedarf an einem Verfahren, das die Gewinnung von MMDA aus MDA mit hoher Reinheit, d. h insbesondere mit einem möglichst geringen Anteil an PMDA, ermöglicht, ohne dass dazu ein großer verfahrenstechnischer (Destillations-)Aufwand betrieben werden müsste. Insbesondere sollte sich das Verfahren möglichst einfach in bestehende Anlagen zur Produktion von MDA integrieren lassen, und das nach Abtrennung des MMDA verbleibende MDA (mit einem notwendigerweise verringertem MMDA-Anteil) sollte weiterhin für die üblichen Einsatzzwecke von MDA geeignet sein.

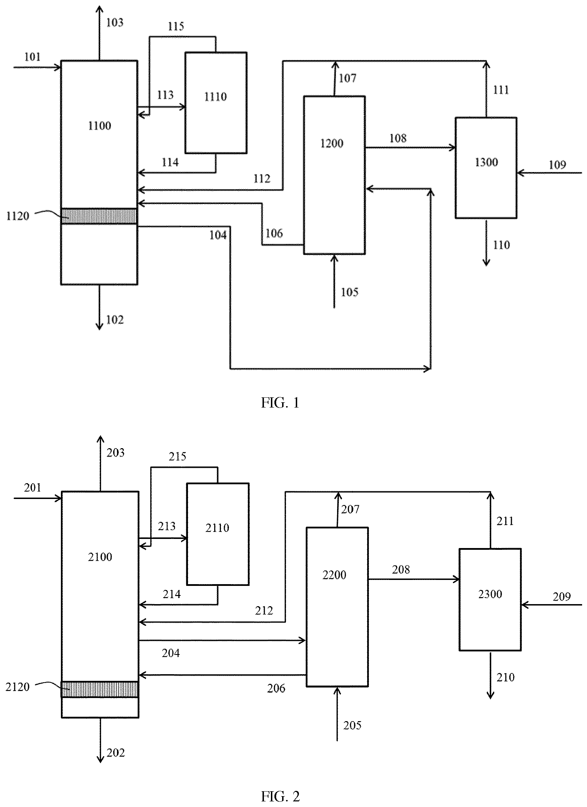

Dem vorstehend Gesagten Rechnung tragend ist ein Gegenstand der vorliegenden Erfindung ein im Folgenden beschriebenes Verfahren zur Herstellung von Di- und Polyaminen der Diphenylmethanreihe. Die mit "X" beginnenden Bezugszeichen in Klammern beziehen sich dabei auf die beigefügten Zeichnungen FIG. 1 bis FIG. 7, wobei "X" für die Nummer der Zeichnung steht. Das erfindungsgemäße Verfahren umfasst die Schritte:
a) Säure-katalysiertes Kondensieren von Anilin und Formaldehyd unter Erhalt eines sauren, Wasser, Di- und Polyamine der Diphenylmethanreihe (MDA) und Anilin enthaltenden Verfahrensprodukts;
b) Neutralisieren des in Schritt a) erhaltenen Verfahrensprodukts und anschließende Trennung in eine organische, MDA und Anilin enthaltende Phase und eine wässrige Phase;
c) optional (und bevorzugt), Waschen der in Schritt b) erhaltenen organischen, MDA und Anilin enthaltenden Phase;
d) destillative Aufarbeitung der in Schritt b) oder Schritt c) erhaltenen organischen, MDA und Anilin enthaltenden Phase (X01) unter Erhalt eines, bezogen auf seine Gesamtmasse, mindestens 25,0 Massen-%, bevorzugt 30,0 Massen-% bis 70,0 Massen-% und besonders bevorzugt 35,0 Massen-% bis 65,0 Massen-%, Polyamine der Diphenylmethanreihe (PMDA) enthaltenden **ersten Produktstroms (X02),** wobei der Rest zu 100 Massen-% mindestens aus den Diaminen der Diphenylmethanreihe (MMDA) besteht, und eines, bezogen auf seine Gesamtmasse, mindestens 95,0 Massen-% (d. h. 95,0 Massen-% bis 100 Massen-%, insbesondere 95,0 Massen-% bis 99,999 Massen-%), bevorzugt mindestens 97,0 Massen-%, besonders bevorzugt mindestens 98,0 Massen-% Diamine der Diphenylmethanreihe (MMDA) enthaltenden **zweiten Produktstroms (X10),** umfassend die Schritte
   - in einer Destillationskolonne, Abtrennen eines Anilin und Wasser enthaltenden Stroms (X03) als Kopfprodukt unter Erhalt des **ersten Produktstroms (X02)** als Sumpfprodukt,
   - in dieser Destillationskolonne oder in einer stromabwärts zu dieser Destillationskolonne angeordneten Vorrichtung, Strippen mit Wasserdampf unter Erhalt eines gasförmigen Anilin, Wasser und MMDA enthaltenden Stroms,
   - partielles Kondensieren des durch das Strippen mit Wasserdampf (X05) erhaltenen gasförmigen, Anilin, Wasser und MMDA enthaltenden Stroms unter Erhalt eines MMDA und Wasser (sowie gegebenenfalls Anilin) enthaltenden flüssigen Stroms und eines gasförmigen Stroms enthaltend Anilin und Wasser (sowie gegebenenfalls MMDA),
   - Trocknen, insbesondere durch Strippen mit einem Inertgas (X09) (vorzugsweise Stickstoff) unter Erwärmen, des durch das partielle Kondensieren erhaltenen MMDA und Wasser (sowie gegebenenfalls Anilin) enthaltenden flüssigen Stroms, insbesondere gefolgt von einer Abkühlung, unter Erhalt des **zweiten Produktstroms (X10).**

Im Sinne der vorliegenden Erfindung meint *"Strippen"* ein physikalisches Trennverfahren, bei dem das zu trennende Stoffgemisch mit einem Gas (dem *"Strippgas")* in Kontakt gebracht wird, um den Trennvorgang zu unterstützen. Im erfindungsgemäßen Schritt d) wird das Abtrennen von Anilin und Wasser durch Strippen mit *Wasserdampf* als Strippgas unterstützt. Dieses Strippen mit Wasserdampf findet erfindungsgemäß entweder *in* der Destillationskolonne statt, in der auch der erste Produktstrom (X02) erhalten wird (indem in diese Destillationskolonne Wasserdampf eingeleitet wird), oder in einer *"stromabwärts zu dieser Destillationskolonne angeordneten Vorrichtung". "Stromabwärts angeordnet"* meint dabei eine solche Verschaltung beider Apparate, dass ein Ausgangsstrom der Destillationskolonne ein Eingangsstrom besagter Vorrichtung zum Wasserdampf-Strippen ist. Hierzu ist es bevorzugt, wie weiter unten anhand von Beispielen für konkrete Kolonnenverschaltungen noch näher ausgeführt wird, die Vorrichtung zum Wasserdampf-Strippen, optional über zwischengeschaltete weitere Apparate wie insbesondere einen Verdampfer, *mit einem Seitenabzug* der Destillationskolonne zu verbinden. Es ist auch möglich, *einen Teil des Sumpfstroms der Destillationskolonne* in die Vorrichtung zum Wasserdampf-Strippen zu führen. Geeignete Vorrichtungen zum Strippen mit Wasserdampf sind dem Fachmann bekannt (Strippkolonnen, insbesondere Packungskolonnen, z. B. Füllkörperkolonnen oder Kolonnen mit strukturierter Packung).

Der *"mindestens 95,0 Massen-%, bevorzugt mindestens 97,0 Massen-%, besonders bevorzugt mindestens 98,0 Massen-% Diamine der Diphenylmethanreihe (MMDA)"* enthaltende **zweite Produktstroms (X10)** kann neben MMDA insbesondere noch geringfügige Anteile an PMDA sowie nicht umgesetztes Anilin, Wasser und gegebenenfalls Nebenkomponenten enthalten.

Es folgt zunächst eine Kurzzusammenfassung verschiedener möglicher Ausführungsformen der Erfindung.

In einer **ersten Ausführungsform** der Erfindung, die mit allen anderen Ausführungsformen kombiniert werden kann, enthält der erste Produktstrom 30,0 Massen-% bis 70,0 Massen-% Polyamine der Diphenylmethanreihe, und der zweite Produktstrom enthält mindestens 97,0 Massen-% Diamine der Diphenylmethanreihe.

In einer **zweiten Ausführungsform** der Erfindung, die eine besondere Ausgestaltung der ersten Ausführungsform ist, enthält der erste Produktstrom 35,0 Massen-% bis 65,0 Massen-% Polyamine der Diphenylmethanreihe, und der zweite Produktstrom enthält mindestens 98,0 Massen-% Diamine der Diphenylmethanreihe.

In einer **dritten Ausführungsform** der Erfindung, die mit allen anderen Ausführungsformen kombiniert werden kann, wird in Schritt a) ein molares Verhältnis von insgesamt eingesetztem Anilin zu insgesamt eingesetztem Formaldehyd von 1,6 oder mehr verwendet.

In einer **vierten Ausführungsform** der Erfindung, die mit allen anderen Ausführungsformen kombiniert werden kann, ist Schritt c) umfasst.

In einer **fünften Ausführungsform** der Erfindung, die mit allen anderen Ausführungsformen kombiniert werden kann, erfolgt das Strippen mit Wasserdampf in der Destillationskolonne.

In einer **sechsten Ausführungsform** der Erfindung, die eine Alternative zur fünften Ausführungsform ist und ansonsten mit allen anderen Ausführungsformen kombiniert werden kann, erfolgt das Strippen mit Wasserdampf in einer stromabwärts zur Destillationskolonne angeordneten Vorrichtung.

In einer **siebten Ausführungsform** der Erfindung, die eine besondere Ausgestaltung der sechsten Ausführungsform ist, wird als stromabwärts zur Destillationskolonne angeordnete Vorrichtung eine Packungskolonne eingesetzt.

In einer **achten Ausführungsform** der Erfindung, die eine besondere Ausgestaltung der sechsten und siebten Ausführungsform ist, ist die stromabwärts zur Destillationskolonne angeordnete Vorrichtung, optional über einen zwischengeschalteten Apparat, mit einem Seitenabzug der Destillationskolonne verbunden.

In einer **neunten Ausführungsform** der Erfindung, die eine besondere Ausgestaltung der achten Ausführungsform ist, wird ein Apparat zwischen die Destillationskolonne und die Vorrichtung geschaltet.

In einer **zehnten Ausführungsform** der Erfindung, die eine besondere Ausgestaltung der neunten Ausführungsform ist, ist der Apparat ein Verdampfer.

In einer **elften Ausführungsform** der Erfindung, die eine besondere Ausgestaltung der sechsten und siebten Ausführungsform ist, wird ein Teil des Sumpfprodukts der Destillationskolonne in die stromabwärts zur Destillationskolonne angeordnete Vorrichtung geleitet.

In einer **zwölften Ausführungsform** der Erfindung, die mit allen anderen Ausführungsformen kombiniert werden kann, wird in Schritt d) das Trocknen durch Strippen mit einem Inertgas unter Erwärmen durchgeführt.

In einer **dreizehnten Ausführungsform** der Erfindung, die eine besondere Ausgestaltung der zwölften Ausführungsform ist, wird in Schritt d) nach dem Trocknen eine Abkühlung durchgeführt.

Die zuvor kurz geschilderten Ausführungsformen und weitere mögliche Ausgestaltungen der Erfindung werden im Folgenden näher erläutert. Dabei können die Ausführungsformen beliebig miteinander kombiniert werden, sofern sich aus dem Zusammenhang nichts anderes ergibt.

In **Schritt** a) des erfindungsgemäßen Verfahrens werden Anilin und Formaldehyd zu MDA unter Verwendung saurer Katalysatoren umgesetzt. Durch die Variation des molaren Verhältnisses von Säure zu Anilin (Protonierungsgrad) und von Anilin zu Formaldehyd (A/F-Verhältnis) können der Anteil des von MMDA und die Isomerenzusammensetzung im MDA in weiten Bereichen eingestellt werden. Erfindungsgemäß erfolgt die Umsetzung in Schritt a) so, dass nicht alles Anilin reagiert, sondern ein Teil des Anilins nicht umgesetzt wird. Bevorzugt wird daher im Rahmen der vorliegenden Erfindung ein molares Verhältnis von insgesamt eingesetztem Anilin zu insgesamt eingesetztem Formaldehyd gleich oder größer als 1,6 gewählt. (Stöchiometrisch wird zur Bildung des Diamins ein molares Verhältnis von insgesamt eingesetztem Anilin zu insgesamt eingesetztem Formaldehyd von 2,0 benötigt; bei den *Poly*aminen ist das Verhältnis kleiner als 2,0. Bevorzugt beträgt das molare Verhältnis von insgesamt eingesetztem Anilin zu insgesamt eingesetztem Formaldehyd maximal 20.)

Bevorzugt wird Formaldehyd in Form einer 20%igen bis 60%igen wässrigen Lösung, bezogen auf die Gesamtmasse der Formaldehydlösung, eingesetzt. Es können aber grundsätzlich auch andere Formaldehydquellen, wie z. B. Paraformaldehyd, Trioxan oder gasförmiges Formaldehyd, verwendet werden. Formaldehyd kann dem erfindungsgemäßen Verfahren demnach in Form von monomerem Formaldehyd und / oder in Form von höheren Homologen, sogenannten Poly(oxymethylen)glykolen, zugeführt werden. Als Katalysatoren werden üblicherweise Brønstedt-Säuren eingesetzt. Bevorzugt ist der Einsatz von HCl, entweder als gasförmiger Chlorwasserstoff (der in Anilin absorbiert wird) oder als Salzsäure, besonders bevorzugt 15%ige bis 36%ige Salzsäure, bezogen auf die Gesamtmasse der Salzsäure. Grundsätzlich können auch andere Säuren als homogene Katalysatoren eingesetzt werden, wie in den eingangs zitierten Literaturstellen beschrieben. Ebenfalls ist die Verwendung von heterogenen, saure Zentren enthaltenden Katalysatoren möglich.

In einer **ersten Variante von Schritt a)** wird Anilin zunächst in Abwesenheit eines sauren Katalysators mit Formaldehyd zur Reaktion gebracht, danach die wässrige Phase abgetrennt und die organische Phase mit dem sauren Katalysator versetzt. Dabei wird das Anilin zunächst mit Formaldehyd in einem molaren Verhältnis Anilin : Formaldehyd von mindestens 1,6 : 1 bevorzugt 2,1 : 1 bis 20 : 1, besonders bevorzugt von 2,1 : 1 bis 4,0 : 1, und bei einer Temperatur von bevorzugt 10 °C bis 150 °C, besonders bevorzugt von 75 °C bis 110 °C, umgesetzt. Dann wird der organischen Phase der saure Katalysator in einem molaren Verhältnis Anilin : saurer Katalysator von bevorzugt 2,0 : 1 bis 100 : 1 (entsprechend einem Protonierungsgrad des Anilins von 50 % bis 1,0 %), besonders bevorzugt 4,0 : 1 bis 20 : 1, bei einer Temperatur von bevorzugt 10 °C bis 150 °C, besonders bevorzugt von 35 °C bis 75 °C zugegeben. Als saurer Katalysator wird bevorzugt Salzsäure, besonders bevorzugt 15%ige bis 36%ige Salzsäure, bezogen auf die Gesamtmasse der Salzsäure, eingesetzt. Die weitere Umsetzung zur Vervollständigung der Reaktion wird im Temperaturbereich von 20 °C bis 220 °C, bevorzugt von 35 °C bis 180 °C durchgeführt.

In einer **zweiten Variante von Schritt a)** werden zunächst Anilin und saurer Katalysator vermischt, bevor Formaldehyd zugesetzt wird. Dabei wird dem Anilin der saure Katalysator in einem molaren Verhältnis Anilin: saurer Katalysator von bevorzugt 2,0 : 1 bis 100 : 1 (entsprechend einem Protonierungsgrad des Anilins von 50 bis 1,0 %), besonders bevorzugt 4,0 : 1 bis 20 : 1, bei einer Temperatur von bevorzugt 10 °C bis 150 °C, besonders bevorzugt von 30 °C bis 100 °C zugegeben. Als saurer Katalysator wird bevorzugt Salzsäure, besonders bevorzugt 15%ige bis 36%ige Salzsäure, bezogen auf die Gesamtmasse der Salzsäure, eingesetzt. Dann wird das Reaktionsgemisch zunächst mit Formaldehyd in einem molaren Verhältnis von mindestens 1,6 : 1, bevorzugt 2,1 : 1 bis 20 : 1, besonders bevorzugt von 2,1 : 1 bis 4,0 : 1, und bei einer Temperatur von bevorzugt 10 °C bis 150 °C, besonders bevorzugt 30 °C bis 140 °C, ganz besonders bevorzugt 35 °C bis 75 °C umgesetzt. Die weitere Umsetzung zur Vervollständigung der Reaktion wird im Temperaturbereich von 20 °C bis 220 °C, bevorzugt von 35 °C bis 180 °C durchgeführt.

In einer **dritten Variante von Schritt a)** wird zunächst eine erste Teilmenge des Anilins mit dem Formaldehyd (z. B. als wässrige Lösung) zur Reaktion gebracht und die wässrige Phase von der organischen Phase getrennt, sowie eine zweite Teilmenge des Anilins mit dem sauren Katalysator (z. B. Salzsäure) vermischt, bevor sie mit der erwähnten organischen Phase zusammengeführt wird. Dabei wird die erste Teilmenge Anilin zunächst mit Formaldehyd in einem molaren Verhältnis Anilin : Formaldhehyd von bevorzugt 1,4 : 1 bis 20 : 1, besonders bevorzugt von 1,4 : 1 und 4,0 : 1, und bei einer Temperatur von bevorzugt von 10 °C bis 150 °C, besonders bevorzugt von 75 °C bis 110 °C, umgesetzt. Der zweiten Teilmenge Anilin wird der saure Katalysator in einem molaren Verhältnis Anilin: saurer Katalysator von bevorzugt 0,5 : 1 bis 10 : 1 (entsprechend einem Protonierungsgrad des Anilins von 200 % bis 10 %), besonders bevorzugt 1,0 : 1 bis 5 : 1, bei einer Temperatur von bevorzugt 10 °C bis 150 °C, besonders bevorzugt 30 °C bis 100 °C zugegeben. Als saurer Katalysator wird bevorzugt Salzsäure, besonders bevorzugt 15%ige bis 36%ige Salzsäure, bezogen auf die Gesamtmasse der Salzsäure, eingesetzt. Dann wird die nach Umsetzung der ersten Teilmenge des Anilins mit dem Formaldehyd erhaltene organische Phase mit der den sauren Katalysator enthaltenen zweiten Teilmenge des Anilins bei einer Temperatur von bevorzugt 10 °C bis 150 °C, besonders bevorzugt 35 °C bis 75 °C zusammengeführt. An dieser Stelle der Umsetzung beträgt das molare Verhältnis Anilin : Formaldehyd 1,6 : 1 bis 20 : 1, besonders bevorzugt von 2,1 : 1 bis 4,0 : 1, und das molare Verhältnis Anilin : saurer Katalysator beträgt bevorzugt 2,0 : 1 bis 100 : 1 (entsprechend einem Protonierungsgrad des Anilins von 50 % bis 1,0 %), besonders bevorzugt 4,0 : 1 bis 20 : 1. Die weitere Umsetzung zur Vervollständigung der Reaktion wird im Temperaturbereich von 20 °C bis 220 °C, bevorzugt von 35 °C bis 180 °C durchgeführt.

Geeignete *Mischvorrichtungen* zur innigen Vermischung der Edukte und Katalysator sind beispielsweise Mischpumpen, Düsen oder statische Mischer. Ferner werden die Edukte in einer geeigneten Reaktionsvorrichtung, wie beispielsweise in Rohrreaktoren, Rührreaktoren und Reaktionskolonnen oder deren Kombinationen, umgesetzt.

Üblicherweise wir die Umsetzung ohne den Einsatz eines weiteren Lösungsmittels durchgeführt. Als Reaktionsmedium dient (überschüssiges) Anilin und das im Reaktionsgemisch enthaltene Wasser. Die Umsetzung vom Anilin mit Formaldehyd kann sowohl kontinuierlich als auch diskontinuierlich, in einem Batch- oder Semibatch-Verfahren erfolgen.

Das in Schritt a) erhaltene MDA-haltige Verfahrensprodukt enthält, bezogen auf das darin enthaltene MDA, bis zu 85 Massen-%, bevorzugt 30 Massen-% bis 70 Massen-% und besonders bevorzugt 35 Massen-% bis 65 Massen-%, MMDA.

In **Schritt b)** des erfindungsgemäßen Verfahrens wird der saure Katalysator durch Zugabe einer Base neutralisiert und das Reaktionsgemisch in eine organische und eine wässrige Phase getrennt. Die organische Phase enthält die Di- und Polyamine der Diphenylmethanreihe (MDA) sowie nicht umgesetztes Anilin. Dabei wird das Reaktionsgemisch enthaltend die Di- und Polyamine der Diphenylmethanreihe ggf. unter Zusatz von Wasser und / oder Anilin neutralisiert. Nach dem Stand der Technik erfolgt die Neutralisation üblicherweise bei Temperaturen von beispielsweise 90 °C bis 120 °C ohne Zusatz weiterer Substanzen. Sie kann aber auch auf einem anderen Temperaturniveau erfolgen, um z. B. den Abbau von störenden Nebenprodukten zu beschleunigen. Als Basen sind beispielsweise geeignet die Hydroxide der Alkali- und Erdalkalielemente. Vorzugsweise kommt wässrige NaOH zur Anwendung. Die zur Neutralisation eingesetzte Base wird bevorzugt in Mengen von größer als 100 %, besonders bevorzugt 105 % bis 120 % der für die Neutralisation des eingesetzten sauren Katalysators stöchiometrisch benötigten Menge eingesetzt (siehe EP 1 652 835 A1). In der Terminologie der vorliegenden Erfindung umfasst der Begriff *Neutralisation* also auch ein "*Über*neutralisieren" bis in den alkalischen Bereich.

Die in Schritt b) erhaltene, organische, Di- und Polyamine der Diphenylmethanreihe (MDA) sowie nicht umgesetztes Anilin enthaltende Phase wird anschließend bevorzugt in **Schritt c)** mit Wasser gewaschen, wobei nach Abscheidung der Waschwasserphase eine von Salzen weitgehend bis vollständig befreite organische Phase umfassend MDA (und überschüssiges Anilin) erhalten wird. Bevorzugt wird dazu wie in DE-A-2549890, Seite 3, beschrieben, verfahren. In der Terminologie der vorliegenden Erfindung umfasst der Begriff *Wäsche* (dem fachmännischen Gebrauch entsprechend) also stets auch die Abtrennung der Waschwasserphase nach erfolgter Vermischung von zu reinigender organischer Phase und Waschwasser.

Auch nach Abtrennung der Waschwasserphase enthält die verbleibende organische, MDA und Anilin enthaltende Phase noch Wasser. Dies ist auf eine Restlöslichkeit von Wasser in der organischen Phase zurückzuführen; außerdem kann es in der Praxis - insbesondere im großtechnischen Maßstab - durchaus vorkommen, dass die Phasentrennung nicht perfekt verläuft und ein (kleiner) Anteil Waschwasserphase in die weitere Aufarbeitung der organischen, MDA und Anilin enthaltenden Phase mitgeschleppt wird. Entsprechendes gilt natürlich für die wässrige Phase nach der Neutralisation, wenn auf die Wäsche verzichtet wird; auch hier verbleibt ein (kleiner) Anteil der wässrigen Phase in der MDA und Anilin enthaltenden organischen Phase.

Die weitere Aufarbeitung der in Schritt b) oder Schritt c) erhaltenen organischen Phase ("Roh-MDA") **in Schritt d)** erfolgt erfindungsgemäß destillativ. In dieser Aufarbeitung werden die beiden Produktströme des erfindungsgemäßen Verfahrens gewonnen, ein MDA-Strom **(erster Produktstrom)** und ein MMDA-Strom **(zweiter Produktstrom).**

Der Schritt d) kann in mehreren verschiedenen Varianten realisiert werden, die weiter unten anhand der beigefügten Zeichnungen näher erläutert werden. Allen Varianten ist gemein, dass aus der aufzuarbeitenden organischen Phase aus Schritt b) oder Schritt c) zunächst in einer Destillationskolonne *ein Anilin und Wasser enthaltender Kopfstrom abgetrennt* wird, wobei der **erste Produktstrom** als Sumpfprodukt dieser Destillationskolonne anfällt. Dieses Sumpfprodukt enthält PMDA in einem Anteil von mindestens 25,0 Massen-%, bevorzugt 30,0 Massen-% bis 70,0 Massen-% und besonders bevorzugt 35,0 Massen-% bis 65,0 Massen-%, bezogen auf die Gesamtmasse des Sumpfprodukts. Der Rest zu 100 Massen-% umfasst MMDA sowie gegebenenfalls hochsiedende Nebenprodukte. Die Zusammensetzung dieses Stromes, insbesondere der MMDA-Anteil und das Verhältnis von PMDA zu MMDA, kann mit dem Fachmann bekannten analytischen Methoden wie insbesondere HPLC oder GC leicht ermittelt werden. Im Allgemeinen ergeben die verschiedenen Methoden - im Rahmen insignifikanter Schwankungen - die gleichen Ergebnisse; im Zweifelsfall ist die Zwecke der vorliegenden Erfindung der mittels HPLC bestimmte MMDA-Anteil maßgeblich. (Zur Bestimmung der Zusammensetzung mittels HPLC wird das zu untersuchende MDA mit einem geeigneten Chromatographen, z. B. mittels eines HPLC 1260 der Firma Agilent, durch Umkehrphasenchromatographie an einer C 18-Säule mittels Gradientenelution mit einem ternären Gemisch aus Methanol, Acetonitril und Wasser unter Verwendung eines UV-Detektors bei einer Wellenlänge von 240 nm in seine Bestandteile getrennt. Die MMDA-Isomeren werden mit Hilfe einer externen Kalibration quantifiziert.)

Diese Destillation erfolgt bevorzugt bei einer Sumpftemperatur im Bereich von 180 °C bis 260 °C, wahlweise erreicht durch einen internen als Erhitzer-Trockner fungierenden Wärmeaustauscher mit dem Zweck der Trocknung (eventuell unterstützt durch das Hindurchleiten eines Inertgases, insbesondere Stickstoff) und einem Kopfdruck im Bereich von 5 mbar_{(abs.)} bis 300 mbar_{(abs.)}. Das dabei erhaltene Sumpfprodukt (erster Produktstrom) enthält wie bereits erwähnt neben dem PMDA im Wesentlichen MMDA und wird bevorzugt abgekühlt auf eine Temperatur im Bereich von 90 °C bis 140 °C. Dieses Gemisch aus PMDA und MMDA (= MDA) wird seiner weiteren Verwendung zugeführt, bevorzugt der aus dem Stand der Technik bekannten Phosgenierung zu MDI. Das in dieser Destillation erhaltene Kopfprodukt besteht im Wesentlichen aus Anilin und Wasser. Bevorzugt wird zumindest ein Teil dieses Anilins wieder in Schritt a) zurückgeführt.

Des Weiteren umfasst der Schritt d) der vorliegenden Erfindung ein *Strippen mit Wasserdampf unter Erhalt eines gasförmigen Anilin, Wasser und MMDA enthaltenden Stroms.* Diese Wasserdampf-Strippung kann *in* der bereits erwähnten Destillationskolonne oder in einer *nachgeschalteten* Vorrichtung durchgeführt werden. In letztgenanntem Fall ist diese Vorrichtung über einen Seitenabzug mit der Destillationskolonne verbunden. Als Vorrichtungen zur Wasserdampf-Strippung in diesem Sinne eignen sich dem Fachmann bekannte Strippkolonnen. Diese werden bevorzugt bei einer Sumpftemperatur im Bereich von 180 °C bis 250 °C und einem Kopfdruck im Bereich von 30 mbar_{(abs)} bis 300 mbar_{(abs.)} betrieben. In jedem Fall fällt ein gasförmiger, Anilin, Wasser und MMDA enthaltender Strom an, der im Allgemeinen eine Temperatur im Bereich von 180 °C bis 250 °C aufweist und unter einem Druck im Bereich von 5 mbar_{(abs.)} bis 300 mbar_{(abs.)} steht.

Der in der Wasserdampf-Strippung anfallende, gasförmiges Anilin, Wasser und MMDA enthaltende Strom wird erfindungsgemäß *partiell kondensiert.* Geeignete technische Einrichtungen hierzu wie Kondensatoren sind dem Fachmann bekannt. Diese Einrichtungen können in Destillationskolonnen integriert oder als eigenständige Apparate ausgelegt sein und werden bevorzugt bei einer Temperatur im Bereich von 120 °C bis 210 °C und unter einem Druck im Bereich von 5 mbar_{(abs.)} bis 300 mbar_{(abs.)} betrieben. Bei der partiellen Kondensation werden ein MMDA und Wasser (sowie gegebenenfalls Anilin) enthaltender flüssiger Strom und ein gasförmiger Strom enthaltend Anilin und Wasser (sowie gegebenenfalls MMDA) erhalten. Dieser gasförmige Strom wird bevorzugt in die Destillation zur Abtrennung von Anilin und Wasser in der weiter oben diskutierten Destillationskolonne geführt.

Zur Gewinnung des **zweiten Produktstroms** wird der so erhaltene MMDA und Wasser enthaltende flüssige Strom *getrocknet.* Dies kann grundsätzlich auf alle dem Fachmann geläufige Weisen geschehen. Erfindungsgemäß bevorzugt ist es, den flüssigen Strom aus der partiellen Kondensation unter Erwärmung, insbesondere auf eine Temperatur im Bereich von 210 °C bis 260 °C, durch Hindurchleiten eines Inertgases, insbesondere Stickstoff, zu strippen. Bevorzugt schließt sich an die Trocknung des flüssigen Stroms (unmittelbar) eine Abkühlung des getrockneten Stroms auf eine Temperatur im Bereich von 90 °C bis 140 °C an. Auf diese Weise wird der **zweite Produktstrom** enthaltend mindestens 95,0 Massen-%, bevorzugt mindestens 97,0 Massen-%, besonders bevorzugt mindestens 98,0 Massen-% MMDA, bezogen auf die Gesamtmasse dieses Stroms, als Kondensat erhalten. Es verbleibt eine Gasphase, die neben Wasser und dem eingesetzten Inertgas auch noch 2,2'-MMDA und 2,4'-MMDA als Leichtsieder enthalten kann und daher gegebenenfalls bevorzugt in die Destillation zur Abtrennung von Anilin und Wasser in der weiter oben diskutierten Destillationskolonne geführt wird. Die Differenz zu 100 Massen-% in diesem zweiten Produktstrom besteht im Wesentlichen aus Wasser, Anilin und PMDA, wobei der PMDA-Anteil des zweiten Produktstroms bevorzugt maximal 4,0 Massen-%, besonders bevorzugt maximal 3,0 Massen-%, ganz besonders bevorzugt maximal 2,0 Massen-%, bezogen auf seine Gesamtmasse, ausmacht. Bevorzugt enthält dieser MMDA-Strom 83,0 Massen-% bis 92,0 Massen-% 4,4'-Diaminodiphenylmethan, 7,0 Massen-% bis 14,0 Massen-% 2,4'-Diaminodiphenylmethan und 0,2 Massen-% bis 0,8 Massen-% 2,2'-Diaminodiphenylmethan, jeweils bezogen auf die Gesamtmasse der MMDA-Isomeren. Die Zusammensetzung des zweiten Produktstroms kann mit den gleichen analytischen Methoden wie für den ersten Produktstrom zuvor beschrieben bestimmt werden.

Dieser erfindungsgemäß als zweiter Produktstrom hergestellte MMDA-Strom eignet sich hervorragend für verschiedene Anwendungen. Bevorzugt sind die folgenden Verwendungsmöglichkeiten dieses MMDA-Stroms:
- Gasphasen- oder Flüssigphasenphosgenierung zu MMDI
- Kernhydrierung zu H₁₂-MMDA
- Härter für Epoxy Lacke und Composites
- Rohstoff für Polyetheretherketone (PEEK)

**Schritt d)** des erfindungsgemäßen Verfahrens wird nachfolgend noch mit Hilfe der beigefügten **Zeichnungen** näher erläutert. In allen Zeichnungen werden Stoffströme mit dreistelligen Ziffern und Apparate mit vierstelligen Ziffern bezeichnet, wobei die erste Ziffer jeweils die Nummer der Abbildung bezeichnet und die folgenden Ziffern für gleiche oder vergleichbare Stoffströme bzw. Apparate gleich sind. Es zeigen:
**FIG. 1** eine mögliche Ausgestaltung des erfindungsgemäßen Verfahrens mit zwei Destillationsschritten in den Destillationskolonnen 1100 und 1200;
**FIG. 2** eine weitere mögliche Ausgestaltung des erfindungsgemäßen Verfahrens mit zwei Destillationsschritten in den Destillationskolonnen 2100 und 2200;
**FIG. 3** eine mögliche Ausgestaltung des erfindungsgemäßen Verfahrens mit einem Destillationsschritt in der Destillationskolonne 3100;
**FIG. 4** eine weitere mögliche Ausgestaltung des erfindungsgemäßen Verfahrens mit einem Destillationsschritt in der Destillationskolonne 4100;
**FIG. 5** eine weitere mögliche Ausgestaltung des erfindungsgemäßen Verfahrens mit zwei Destillationsschritten in den Destillationskolonnen 5100 und 5200;
**FIG. 6** eine weitere mögliche Ausgestaltung des erfindungsgemäßen Verfahrens mit zwei Destillationsschritten in den Destillationskolonnen 6100 und 6200;
   und
**FIG. 7** eine weitere mögliche Ausgestaltung des erfindungsgemäßen Verfahrens mit zwei Destillationsschritten in den Destillationskolonnen 7100 und 7200.

Übersicht über die Apparate und wichtigsten Stoffströme (X = Nummer der Zeichnung):

| **Apparate** | | **Stoffströme** | |
|---|---|---|---|
| X100 | Destillationskolonne (optional mit einem internen als Erhitzer-Trockner fungierenden Wärmeaustauscher X120 im unteren Bereich; ein nicht dargestellter als Kühler betriebener Wärmeaustauscher befindet sich in der Kolonne im unteren Bereich derselben, wenn der Erhitzer-Trockner vorhanden ist, unterhalb von diesem, oder ist der Kolonne nachgeschaltet) | X01 | Roh-MDA (organische, MDA und Anilin enthaltende Phase, Eingangsstrom der destillativen Aufarbeitung) |
| X110 | Verdampfer | X02 | Sumpfprodukt der Destillationskolonne X100 (**erster Produktstrom**) |
| X200 | Destillationskolonne (Strippkolonne) | X03 | Kopfprodukt (Brüden) der Destillationskolonne X100 enthaltend Anilin und Wasser |
| X300 | Erhitzer-Kühler (aufweisend einen als Erhitzer betriebenen Wärmeaustauscher im oberen und einen als Kühler betriebenen Wärmeaustauscher im unteren Bereich) | X04 | Als Seitenabzug der Destillationskolonne X100 entnommener Strom enthaltend PMDA, MMDA, Anilin und Wasser |
| | | X05 | Wasserdampf |
| | | X06 | Im unteren Bereich der Strippkolonne X200 abgezogenes MMDA-haltiges Gemisch |
| | | X07 | Brüden der Strippkolonne X200 enthaltend Anilin und Wasser |
| | | X08 | MMDA und Wasser sowie ggf. Anilin enthaltender flüssiger Strom |
| | | X09 | Inertgas (insb. Stickstoff) |
| | | X10 | Nach Abkühlen erhaltener MMDA-Strom (**zweiter Produktstrom**) |
| | | X11 | Brüden des Erhitzer-Kühlers X300 |
| | | X12 | Vereinigte Brüden der Strippkolonne X200 und des Erhitzer-Kühlers X300 |
| | | X13 | Zulaufstrom des Verdampfers X110 |
| | | X14 | Sumpfstrom des Verdampfers X110 |
| | | X15 | Brüden des Verdampfers X110 |

**FIG. 1** stellt eine mögliche Ausführungsform des erfindungsgemäßen Verfahrens dar:
Ein MDA-Strom 101 (die in Schritt b) oder Schritt c) erhaltene organische Phase) wird in eine Destillationskolonne 1100 geleitet. Am Kopf dieser Kolonne wird der Strom 103 enthaltend Anilin und Wasser abgezogen. Die Destillationskolonne 1100 verfügt im unteren Bereich über einen als Erhitzer-Trockner fungierenden Wärmeaustauscher 1120 zur Erzeugung des ersten Produktstroms, der im Sumpfals Strom 102 abgezogen wird. Ein MMDA enthaltendes Gemisch 104 wird unterhalb des Erhitzer-Trockners 1120 als Seitenstrom abgezogen und seitlich in eine Strippkolonne 1200 eingespeist. Die Destillationskolonne 1100 verfügt über einen Verdampfer 1110, welcher über einen Seitenabzug 113, der unterhalb der Zuführung des Stromes 101 liegt, aus der Destillationskolonne gespeist wird. Sumpfaustrag 114 und Brüdenstrom 115 des Verdampfers werden in die Destillationskolonne 1100 zurückgeführt; und zwar der Brüdenstrom 115 unterhalb des Seitenabzugs 113 und der Sumpfstrom 114 oberhalb des Seitenabzugs 104 und unterhalb des Brüdenstroms 115.

Wasserdampf 105 wird von unten nach oben durch die Strippkolonne 1200 geführt. Im unteren Bereich der Strippkolonne 1200 wird ein MMDA- und PMDA-haltiges Gemisch 106 abgezogen und in die Kolonne 1100 oberhalb des Erhitzer-Trockners und unterhalb des Sumpfstromes 114 zurückgeführt. Im oberen Bereich der Strippkolonne 1200 befindet sich ein Kondensator (nicht gezeigt) zur partiellen Kondensation des nach oben steigenden Gasgemisches. Am Kopf der Strippkolonne 1200 werden die nicht kondensierten Anilin- und Wasserdampf-haltigen Brüden 107 (die auch noch MMDA enthalten) abgezogen. In einer unterhalb des Kondensators angeordneten Seitenentnahme der Strippkolonne 1200 oberhalb der Zuführung des Stromes 104 wird ein flüssiger Strom 108 umfassend Anilin, Wasser und MMDA entnommen. Dieser Strom 108 wird im oberen Bereich des Erhitzer-Kühlers 1300 unter Durchleiten eines Inertgases 109 (insbesondere Stickstoff) unter Erwärmen trocken gestrippt. Die Zufuhr des Inertgases 109 erfolgt seitlich unterhalb der Zuführung des Stromes 108. Die auf diese Weise getrocknete Flüssigphase wird im unteren Bereich des Erhitzer-Kühlers 1300 abgekühlt und am Sumpfablauf als **zweiter Produktstrom** abgeführt. Der dem Erhitzer-Kühler entnommene Brüdenstrom 111 umfasst Wasser, das eingesetzte Inertgas (insbesondere Stickstoff) und MMDA (sowie gegebenenfalls nicht mit Strom 107 abgetrenntes Anilin). Die Brüdenströme aus 1200 und 1300 werden zu Strom 112 vereinigt. Dieser Strom 112 wird der Kolonne 1100 oberhalb des Stromes 106 und unterhalb des Stromes 114 zugeführt und dient insbesondere aufgrund seines hohen Wasserdampfanteils ebenfalls als Strippgas.

**FIG. 2** stellt eine weitere mögliche Ausführungsform des erfindungsgemäßen Verfahrens dar:
In der Variante gemäß FIG. 2 wird im Gegensatz zur Variante gemäß FIG. 1 das MMDA enthaltende Gemisch 204 **oberhalb** des Erhitzer-Trockners 2120 der Destillationskolonne 2100 abgezogen (was einen im Vergleich zu Strom 204 erhöhten Wassergehalt zur Folge hat). Das im unteren Bereich der Strippkolonne abgezogene MMDA- und PMDA-haltige Gemisch 206 wird in die Kolonne 2100 oberhalb des Erhitzer-Trockners zurückgeführt.

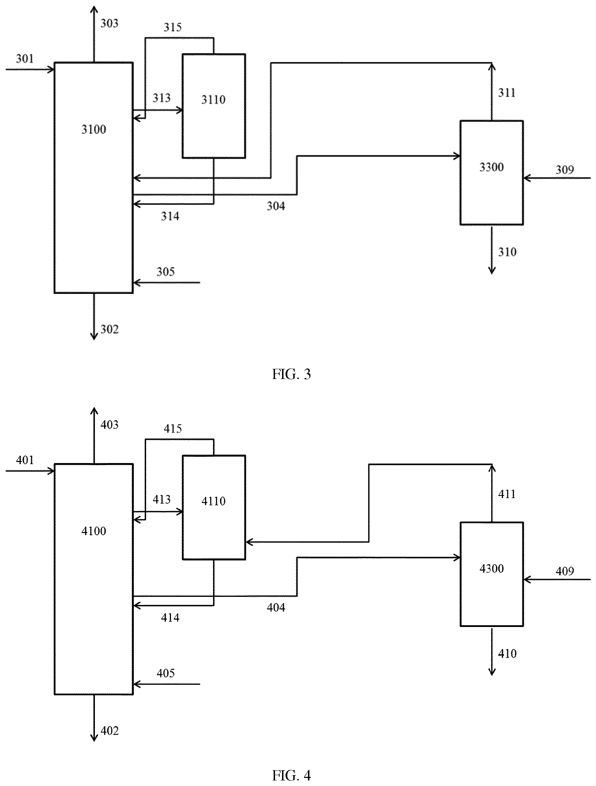

**FIG. 3** stellt eine weitere mögliche Ausführungsform des erfindungsgemäßen Verfahrens dar:
In dieser Variante erfolgt das Strippen mit Wasserdampf 305 *in* der Destillationskolonne 3100, und auf eine zusätzliche Strippkolonne kann daher verzichtet werden. Im Gegensatz zu den Varianten gemäß FIG. 1 und 2 wird hier der Sumpfstrom 314 des Verdampfers 3110 unterhalb des Seitenabzugs 304, der seinerseits unterhalb des Brüdenstroms 311 angeordnet ist, in die Destillationskolonne 3100 zurückgeführt. In dieser Variante ist es möglich, oberhalb des Seitenabzugs 304 einen Kondensator zur partiellen Verflüssigung der aufsteigenden Gasphase vorzusehen, sodass der Strom 304 flüssig entnommen wird. In diesem Fall fungiert der Apparat 3300 als Erhitzer-Kühler und wird betrieben wie in den Varianten gemäß FIG. 1 und 2. Es ist aber auch möglich, den Strom 304 der Destillationskolonne 3100 gasförmig zu entnehmen und die partielle Kondensation außerhalb dieser Destillationskolonne durchzuführen. In diesem Fall muss dann der Strom 304 vor der Trocknung durch Inertgasstrippung einen Kondensator (nicht gezeigt) durchlaufen, der entweder außerhalb von 3300 oder in 3300 oberhalb des als Erhitzer betriebenen Wärmeaustauschers angeordnet ist.

**FIG. 4** stellt eine weitere mögliche Ausführungsform des erfindungsgemäßen Verfahrens dar:
Diese Variante entspricht der Variante gemäß FIG. 3 mit dem Unterschied, dass der Brüdenstrom 411 nicht in die Destillationskolonne 4100, sondern in den Verdampfer 4110 geführt wird.

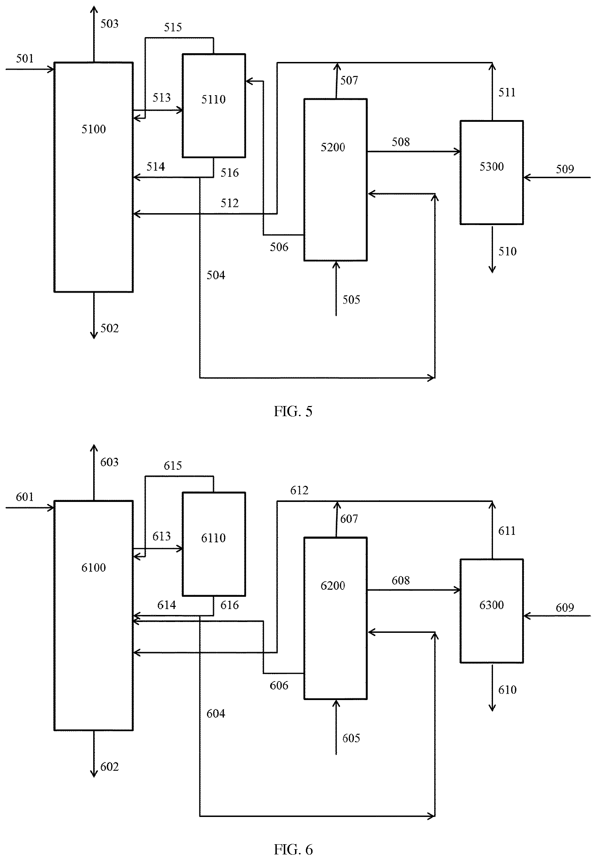

**FIG. 5** stellt eine weitere mögliche Ausführungsform des erfindungsgemäßen Verfahrens dar:
In dieser Variante erfolgt das Strippen mit Wasserdampf 505 wie in den Varianten gemäß FIG. 1 und 2 in einer Stippkolonne 5200. Im Unterschied zu diesen beiden Varianten stammt hier der Zulaufstrom für die Strippkolonne 504 aus dem Sumpfablauf des Verdampfers 5110, von dem nur ein Teil als Strom 514 in die Destillationskolonne 5100 zurückgeführt wird. Anders ausgedrückt ist in dieser Variante die Strippkolonne 5200 über den "zwischengeschalteten" Verdampfer 5110 mit einem Seitenabzug der Destillationskolonne 5100, nämlich 513, verbunden. Das MMDA- und PMDAenthaltende Gemisch 506 wird in den Verdampfer 5110 geführt.

**FIG. 6** stellt eine weitere mögliche Ausführungsform des erfindungsgemäßen Verfahrens dar:
Die Variante gemäß FIG. 6 entspricht der aus FIG. 5 mit dem Unterschied, dass das MMDA- und PMDA-haltige Gemisch 606 nicht in den Verdampfer 6110, sondern, analog der Variante gemäß FIG. 1, zwischen Strom 614 und Strom 612 in die Destillationskolonne 6100 geführt wird.

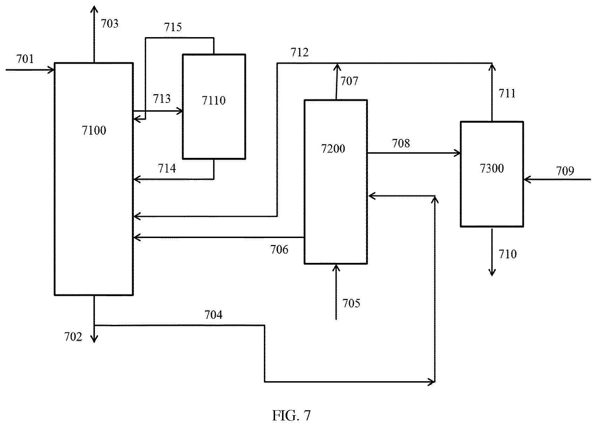

**FIG. 7** stellt eine weitere mögliche Ausführungsform des erfindungsgemäßen Verfahrens dar:
Im Unterschied zu den vorigen Varianten wird hier die Strippkolonne (7200) nicht aus einem Seitenstrom der Destillationskolonne (7100), sondern aus einem aus dem Sumpfstrom der Destillationskolonne abgezweigten Teilstrom (704) gespeist. Ansonsten entspricht die Kolonnenverschaltung der Ausführungsform gemäß FIG. 1.

## Patentansprüche

1. Verfahren zur Herstellung von Di- und Polyaminen der Diphenylmethanreihe, umfassend die Schritte:
a) Säure-katalysiertes Kondensieren von Anilin und Formaldehyd unter Erhalt eines sauren, Wasser, Di- und Polyamine der Diphenylmethanreihe (MDA) und Anilin enthaltenden Verfahrensprodukts;
b) Neutralisieren des in Schritt a) erhaltenen Verfahrensprodukts und anschließende Trennung in eine organische, MDA und Anilin enthaltende Phase und eine wässrige Phase;
c) optional, Waschen der in Schritt b) erhaltenen organischen, MDA und Anilin enthaltenden Phase;
d) destillative Aufarbeitung der in Schritt b) oder Schritt c) erhaltenen organischen, MDA und Anilin enthaltenden Phase unter Erhalt eines, bezogen auf seine Gesamtmasse, mindestens 25,0 Massen-% Polyamine der Diphenylmethanreihe enthaltenden ersten Produktstroms, wobei der Rest zu 100 Massen-% mindestens aus den Diaminen der Diphenylmethanreihe besteht, und eines, bezogen auf seine Gesamtmasse, mindestens 95,0 Massen-% Diamine der Diphenylmethanreihe (MMDA) enthaltenden zweiten Produktstroms, umfassend die Schritte
• in einer Destillationskolonne, Abtrennen eines Anilin und Wasser enthaltenden Stroms als Kopfprodukt unter Erhalt des ersten Produktstroms als Sumpfprodukt,
• in dieser Destillationskolonne oder in einer stromabwärts zu dieser Destillationskolonne angeordneten Vorrichtung, Strippen mit Wasserdampf unter Erhalt eines gasförmigen Anilin, Wasser und MMDA enthaltenden Stroms,
• partielles Kondensieren des durch das Strippen mit Wasserdampf erhaltenen gasförmigen, Anilin, Wasser und MMDA enthaltenden Stroms unter Erhalt eines MMDA und Wasser enthaltenden flüssigen Stroms und eines gasförmigen Stroms enthaltend Anilin und Wasser,
• Trocknen des durch das partielle Kondensieren erhaltenen MMDA und Wasser enthaltenden flüssigen Stroms unter Erhalt des zweiten Produktstroms.

2. Verfahren gemäß Anspruch 1, bei welchem der erste Produktstrom 30,0 Massen-% bis 70,0 Massen-% Polyamine der Diphenylmethanreihe und der zweite Produktstrom mindestens 97,0 Massen-% Diamine der Diphenylmethanreihe enthält.

3. Verfahren gemäß Anspruch 2, bei welchem der erste Produktstrom 35,0 Massen-% bis 65,0 Massen-% Polyamine der Diphenylmethanreihe und der zweite Produktstrom mindestens 98,0 Massen-% Diamine der Diphenylmethanreihe enthält.

4. Verfahren gemäß einem der vorstehenden Ansprüche, bei welchem in Schritt a) ein molares Verhältnis von insgesamt eingesetztem Anilin zu insgesamt eingesetztem Formaldehyd von 1,6 oder mehr gewählt wird.

5. Verfahren gemäß einem der vorigen Ansprüche, bei welchem Schritt c) umfasst ist.

6. Verfahren gemäß einem der Ansprüche 1 bis 5, bei welchem das Strippen mit Wasserdampf in der Destillationskolonne erfolgt.

7. Verfahren gemäß einem der Ansprüche 1 bis 5, bei welchem das Strippen mit Wasserdampf in einer stromabwärts zur Destillationskolonne angeordneten Vorrichtung erfolgt.

8. Verfahren gemäß Anspruch 7, bei welchem die stromabwärts zur Destillationskolonne angeordnete Vorrichtung eine Packungskolonne ist.

9. Verfahren gemäß einem der Ansprüche 7 oder 8, bei welchem die stromabwärts zur Destillationskolonne angeordnete Vorrichtung, optional über einen zwischengeschalteten Apparat, mit einem Seitenabzug der Destillationskolonne verbunden ist.

10. Verfahren gemäß Anspruch 9, bei welchem ein Apparat zwischen die Destillationskolonne und die Vorrichtung geschaltet ist.

11. Verfahren gemäß Anspruch 10, bei welchem der Apparat ein Verdampfer ist.

12. Verfahren gemäß Anspruch 7 oder 8, bei welchem ein Teil des Sumpfprodukts der Destillationskolonne in die stromabwärts zur Destillationskolonne angeordnete Vorrichtung geleitet wird.

13. Verfahren gemäß einem der vorstehenden Ansprüche, bei welchem in Schritt d) das Trocknen durch Strippen mit einem Inertgas unter Erwärmen durchgeführt wird.

14. Verfahren gemäß Anspruch 13, bei welchem in Schritt d) nach dem Trocknen eine Abkühlung durchgeführt wird.

15. Verfahren gemäß Anspruch 13 oder 14, bei welchem das zum Trocknen eingesetzte Inertgas Stickstoff umfasst.

## Claims

1. Process for the preparation of di- and polyamines of the diphenylmethane series, comprising the steps:
a) subjecting aniline and formaldehyde to acid catalyzed condensation so as to obtain an acidic process product containing water, di- and polyamines of the diphenylmethane series (MDA), and aniline;
b) neutralizing the process product obtained in step a) followed by separation into an organic phase containing MDA and aniline and an aqueous phase;
c) optionally, washing the organic phase containing MDA and aniline obtained in step b) ;
d) distilling the organic phase containing MDA and aniline obtained in step b) or step c) so as to obtain a first product stream containing, in relation to its total mass, at least 25.0 mass-% of polyamines of the diphenylmethane series, wherein the remainder to 100 mass-% consists at least of the diamines of the diphenylmethane series, and a second product stream containing, in relation to its total mass, at least 95.0 mass-% of diamines of the diphenylmethane series (MMDA), the process comprising the steps
• in a distillation column, separating off a stream containing aniline and water as head product so as to obtain the first product stream as sump product,
• in said distillation column or in an apparatus arranged downstream of said distillation column, performing a stripping with steam so as to obtain a gaseous stream containing aniline, water and MMDA,
• partially condensing the gaseous stream containing aniline, water and MMDA obtained by the stripping with steam so as to obtain a liquid stream containing MMDA and water and a gaseous stream containing aniline and water,
• drying the liquid stream containing MMDA and water obtained by the partial condensation so as to obtain the second product stream.

2. Process according to Claim 1, in which the first product stream contains 30.0 mass-% to 70.0 mass-% of polyamines of the diphenylmethane series and the second product stream contains at least 97.0 mass-% of diamines of the diphenylmethane series.

3. Process according to Claim 2, in which the first product stream contains 35.0 mass-% to 65.0 mass-% of polyamines of the diphenylmethane series and the second product stream contains at least 98.0 mass-% of diamines of the diphenylmethane series.

4. Process according to one of the preceding claims, in which in step a) a molar ratio of the total aniline used to the total formaldehyde used of 1.6 or more is selected.

5. Process according to one of the preceding claims, in which step c) is included.

6. Process according to one of Claims 1 to 5, in which the stripping with steam is performed in the distillation column.

7. Process according to one of Claims 1 to 5, in which the stripping with steam is performed in an apparatus arranged downstream of the distillation column.

8. Process according to Claim 7, in which the apparatus arranged downstream of the distillation column is a packed column.

9. Process according to one of Claims 7 or 8, in which the apparatus arranged downstream of the distillation column is connected to a side drain of the distillation column, optionally via an intermediate piece of equipment.

10. Process according to Claim 9, in which a piece of equipment is connected between the distillation column and the apparatus.

11. Process according to Claim 10, in which the piece of equipment is an evaporator.

12. Process according to Claim 7 or 8, in which some of the sump product of the distillation column is conducted into the apparatus arranged downstream of the distillation column.

13. Process according to one of the preceding claims, in which in step d) the drying by stripping is performed with an inert gas under heating.

14. Process according to Claim 13, in which in step d) a cooling is performed after the drying.

15. Process according to Claim 13 or 14, in which the inert gas used for drying comprises nitrogen.

## Revendications

1. Procédé pour la préparation de diamines et de polyamines de la série des diphénylméthanes, comprenant les étapes :
a) condensation, catalysée par un acide, d'aniline et de formaldéhyde avec obtention d'un produit de procédé acide, contenant de l'eau, des diamines et des polyamines de la série des diphénylméthanes (MDA) et de l'aniline ;
b) neutralisation du produit de procédé obtenu dans l'étape a) et séparation consécutive en une phase organique, contenant des MDA et de l'aniline, et en une phase aqueuse ;
c) éventuellement lavage de la phase organique, contenant des MDA et de l'aniline, obtenue dans l'étape b) ;
d) traitement par distillation de la phase organique, contenant des MDA et de l'aniline, obtenue dans l'étape b) ou dans l'étape c) avec obtention d'un premier flux produit contenant, par rapport à sa masse totale, au moins 25,0% en masse de polyamines de la série des diphénylméthanes, le reste jusqu'à 100% en masse étant constitué au moins par les diamines de la série des diphénylméthanes, et d'un deuxième flux produit contenant, par rapport à sa masse totale, au moins 95,0% en masse de diamines de la série des diphénylméthanes (MMDA), comprenant les étapes
• de séparation, dans une colonne de distillation, d'un flux contenant de l'aniline et de l'eau comme produit de tête avec obtention du premier flux produit comme produit de fond,
• d'extraction, dans cette colonne de distillation ou dans un dispositif disposé en aval de cette colonne de distillation, à l'aide de vapeur d'eau avec obtention d'un flux gazeux contenant de l'aniline, de l'eau et des MMDA,
• de condensation partielle du flux gazeux, contenant de l'aniline, de l'eau et des MMDA, obtenu par extraction à la vapeur d'eau, avec obtention d'un flux liquide contenant des MMDA et de l'eau et d'un flux gazeux contenant de l'aniline et de l'eau,
• de séchage du flux liquide, contenant des MMDA et de l'eau, obtenu par la condensation partielle avec obtention du deuxième flux produit.

2. Procédé selon la revendication 1, dans lequel le premier flux produit contient 30,0% en masse à 70,0% en masse de polyamines de la série des diphénylméthanes et le deuxième flux produit contient au moins 97,0% en masse de diamines de la série des diphénylméthanes.

3. Procédé selon la revendication 2, dans lequel le premier flux produit contient 35,0% en masse à 65,0% en masse de polyamines de la série des diphénylméthanes et le deuxième flux produit contient au moins 98,0% en masse de diamines de la série des diphénylméthanes.

4. Procédé selon l'une quelconque des revendications précédentes, dans lequel on choisit, dans l'étape a), un rapport molaire de l'aniline utilisée au total au formaldéhyde utilisé au total de 1,6 ou plus.

5. Procédé selon l'une quelconque des revendications précédentes, dans lequel l'étape c) est comprise.

6. Procédé selon l'une quelconque des revendications 1 à 5, dans lequel l'extraction à la vapeur d'eau est réalisée dans la colonne de distillation.

7. Procédé selon l'une quelconque des revendications 1 à 5, dans lequel l'extraction à la vapeur d'eau est réalisée dans un dispositif disposé en aval de la colonne de distillation.

8. Procédé selon la revendication 7, dans lequel le dispositif disposé en aval de la colonne de distillation est une colonne à garnissages.

9. Procédé selon l'une quelconque des revendications 7 ou 8, dans lequel le dispositif disposé en aval de la colonne de distillation est relié, éventuellement via d'un appareil intermédiaire, à un soutirage latéral de la colonne de distillation.

10. Procédé selon la revendication 9, dans lequel un appareil est interposé entre la colonne de distillation et le dispositif.

11. Procédé selon la revendication 10, dans lequel l'appareil est un évaporateur.

12. Procédé selon la revendication 7 ou 8, dans lequel une partie du produit de fond de la colonne de distillation est guidée dans le dispositif disposé en aval de la colonne de distillation.

13. Procédé selon l'une quelconque des revendications précédentes, dans lequel le séchage dans l'étape d) est effectué par extraction à l'aide d'un gaz inerte tout en chauffant.

14. Procédé selon la revendication 13, dans lequel un refroidissement est réalisé après le séchage dans l'étape d) .

15. Procédé selon la revendication 13 ou 14, dans lequel le gaz inerte utilisé pour le séchage comprend de l'azote.
